# EUROPEAN PATENT SPECIFICATION

(11) **EP 3 151 684 B1**
(45) Date of publication and mention of the grant of the patent: **26.09.2018**
(21) Application number: 15720974.3
(22) Date of filing: 04.05.2015
(51) Int. Cl.: A23L 27/30, A23L 2/60

(54) **FLAVORED FOOD AND BEVERAGE PRODUCTS**
AROMATISIERTE LEBENSMITTEL- UND GETRÄNKEPRODUKTE
PRODUITS D'ALIMENTS ET DE BOISSONS AROMATISÉS

(30) Priority: 04.05.2014 US 201461988270 P
(43) Date of publication of application: 12.04.2017
(73) Proprietor: Firmenich SA, 1211 Geneva 8 (CH); Interquim, S.A., 08029 Barcelona (ES)
(72) Inventor: DELATTRE, Maxime, CH-1211 Geneva 8 (CH); LOU, Long In, Plainsboro, NJ 08536 (US); SKIFF, Ronald H., Plainsboro, New Jersey 08536 (US); CRESPO MONTERO, Francisco Javier, E-08029 Barcelona (ES); D'HOORE, Tom Nelly Aimé, E-08029 Barcelona (ES)
(74) Representative: Rupp, Christian
(86) International application number: PCT/EP2015/059756
(87) International publication number: WO 2015/169769

(56) References cited:
- EP-A1- 2 923 584
- EP-A2- 1 258 200
- WO-A1-99/21548
- WO-A1-2013/008875
- JP-B2- 5 290 389
- US-A1- 2013 136 839

## Description

### FIELD

The inventions described herein have use in foods and beverages, particularly those that rely on sweeteners and other flavor components and more particularly those that might rely on compounds and ingredients found in nature.

### BACKGROUND

Naringenin (2,3-Dihydro-5,7-dihydroxy-2(-4-hydroxyphenyl)-4H-1-bemzopyran-4-one) has been reported for use as an antioxidant and therapeutic for example in dietary supplements, in tea, in a cosmetic formulation and in a pharmaceutical product. Naringenin has further been reported for example as a bitter blocker in coffee.

US 2013/0136839 discloses the use of Naringenin for improving the taste of a sweetener.

### SUMMARY

Provided herein is a method of enhancing the sweetness of a sweetener in a food or beverage product comprising adding Naringenin to the product in an amount of 30 ppm to 200 ppm by weight of the total weight of the product wherein the Naringenin does not block the bitter taste of the product when compared to the food or beverage without Naringenin.

Also provided herein is a food or beverage product comprising Naringenin in an amount of from 30 to 200 ppm, by weight, of the total weight of the product, and a sweetener wherein the Naringenin does not block the bitter taste of the product.

### Brief Description of the Drawings

**Figure 1** shows the effects of 125 ppm Naringenin in sucrose sweetened water.
**Figure 2** shows the effects of 175 ppm Naringeninin sucrose sweetened water.
**Figure 3** shows the effects of 175 ppm Naringeninin in a sucrose sweetened sample.
**Figure 4** shows the effects of 125 ppm Naringenin in a sucrose sweetened sample
**Figure 5** shows the effects of 125 ppm and 150 ppm Naringenin in a high fructose corn syrup (HFCS) solution.
**Figure 6** shows the effects of 10, 50, 100 and 200 ppm Naringenin in a sucrose solution.
**Figure 7** shows the effects of 50, 100 and 150 ppm Naringenin in a sucrose solution.
**Figure 8** shows the effects of 100 ppm Naringenin in an orange juice based soft drink.
**Figure 9** shows the effects of 10, 25 and 50 ppm Naringenin in a Reb A sweetened sample.
**Figure 10** shows the effects of 10, 25, 50 ppm, 75. 100 and 150 ppm Naringenin in a Reb A sweetened sample
**Figure 11** shows the effects of 10, 25, 50 ppm, 75. 100 and 150 ppm Naringenin in a SG95 (stevia glycosides) sweetened sample

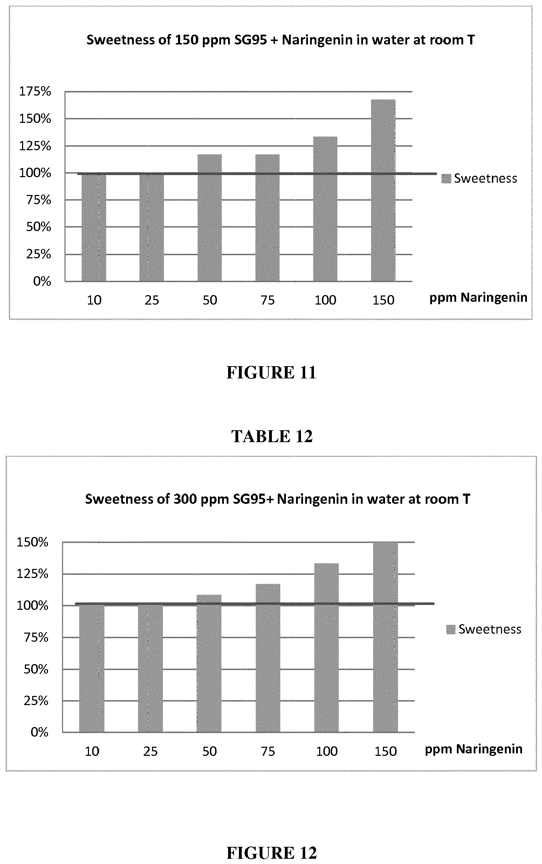

**Figure 11** shows the effects of 10, 25, 50 ppm, 75. 100 and 150 ppm Naringenin in a SG95 (stevia glycosides) sweetened sample.

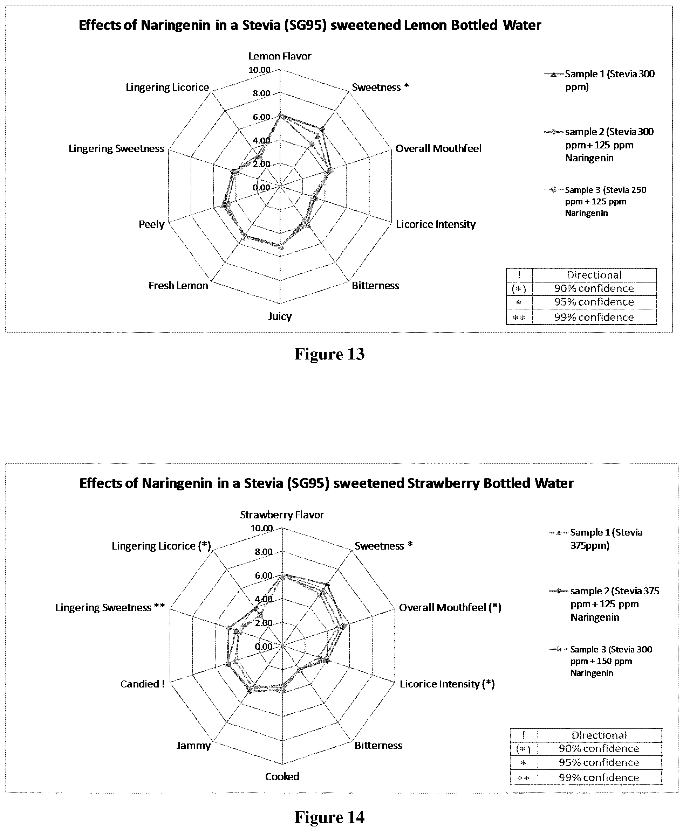

**Figure 13** shows the effects of Naringenin in stevia sweetened lemon flavored bottled water.
**Figure 14** shows the effects of Naringenin in stevia sweetened strawberry flavored bottled water.

### DETAILED DESCRIPTION

For the descriptions herein and the appended claims, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

In one embodiment, Naringenin is provided in a food or beverage in an amount of 50 ppm to 200 ppm by weight of the total weight of the food or beverage, particularly in combination with sucrose or fructose more particularly in combination with 5% sucrose or fructose, by weight of the total weight of the food or beverage.

In one embodiment, Naringenin is provided in a food or beverage in an amount of 75 ppm to 200 ppm by weight of the total weight of the food or beverage.

In a further embodiment, Naringenin is provided in a food or beverage in an amount of 125 ppm to 200 ppm by weight of the total weight of the food or beverage.

In another embodiment, Naringenin is provided in a food or beverage in an amount of 125 to ppm to 175 ppm by weight of the total weight of the food or beverage.

In another embodiment, Naringenin is provided in a food or beverage in an amount of 30 ppm to 175 ppm by weight of the total weight of the food or beverage.

In another embodiment, Naringenin is provided in a food or beverage in an amount of 30 ppm to 100 ppm by weight of the total weight of the food or beverage.

In another embodiment, Naringenin is provided in a food or beverage in an amount of 30 ppm to 75 ppm by weight of the total weight of the food or beverage.

In another embodiment, Naringenin is provided in a food or beverage in an amount of 30 ppm to 50 ppm by weight of the total weight of the food or beverage.

It has been discovered that Naringenin, when tasted in water or in a model beverage solution of water and/or citric acid, it does not contain (for the most part) sweetness on its own. However, when combined with others sweeteners, Naringenin provides sweetness enhancement to composition comprising a sweetener. In some embodiments, Naringenin provides a sweetness equivalent to 1-2°brix of sucrose equivalents. Unexpectedly, it was found that Naringenin did not reduce the bitterness of some of the formulations tested herein. Further, it was found in one formulation that Naringenin actually added bitterness.

In another embodiment, the methods and compositions provided herein can be provided in the substantial absence of a bitter substance, that is provided in an amount below, where the bitter substance confers a bitter taste to a product or beverage. In a particular embodiment, the compositions and methods provided herein are provided in the absence of a bitter substance. A bitter substance may be selected from the group consisting of xanthine alkaloids (for example caffeine, theobromine), quinoline derivatives (for example quinine), polyphenols (for example catechols, flavonols, y-oryzanol, hesperitin), pharmaceutically active compounds (for example fluoroquinolone antibiotics, aspirin, ∼-lactam antibiotics, ambroxol, paracetamol, aspirin, guaifenesin), denatonium benzoate, sucralose octaacetate, potassiumchloride, magnesium salts, urea, bitter amino acids (for example tryptophan) and bitter peptide fragments (for example having a terminal leucine or isoleucine radical).

In another embodiment, Naringenin enhances the flavor profile of a food or beverage, particularly candied intensity and herbal intensity in orange and strawberry flavor bottled water.

In another embodiment, Naringenin enhances the overall mouth-feel of a food or beverage.

In another embodiment, a food or beverage provided herein is not a tea or coffee product, more particularly the food or beverage provided herein is not a coffee food or beverage.

In another embodiment, the sweetener provided herein is selected from the group consisting common saccharide sweeteners, e.g., sucrose, fructose (e.g., D-fructose),glucose (e.g., D-glucose); sweetener compositions comprising natural sugars, such as corn syrup (including high fructose corn syrup) or other syrups or sweetener concentrates derived from natural fruit and vegetable sources; semisynthetic "sugar alcohol" sweeteners such as erythritol, isomalt, lactitol, mannitol, sorbitol, xylitol, maltodextrin, glycerol, threitol, arabitol, ribitol, and dulcitol; artificial sweeteners such as miraculin, aspartame, superaspartame, saccharin, saccharin-sodium salt, acesulfame-K, cyclamate, sodium cyclamate, and alitame; other sweeteners such as trehalose, melizitose, melibiose, raffinose, palatinose, lactulose, cyclamic acid, mogroside, tagatose (e.g., D-tagatose), maltose, galactose (e.g., D-galactose), L-rhamnose, D-sorbose, maunose (e.g., D-maunose), lactose, L-arabinose,D-ribose, D-glyceraldehyde, curculin, brazzein, mogroside, Neohesperidin dihydrochalcone (NHDC), neotame D-tryptophan, D-Ieucine, D-threonine, glycine, D-asparagine, D-phenylalanine, L-proline, maltitol, hydrogenated glucose syrup (HGS), magap, sucralose, lugduname, sucrononate, sucrooctate, monatin, phyllodulcin, hydrogenated starch hydrolyzate (HSH), stevioside, rebaudioside A, rebaudioside D, rebadioside M, and other sweet Stevia based glycosides, lo han guo, thaumatin, monellin, carrelameand amd other guanidine-based sweeteners. Particularly, the sweetener is a high potency sweetener, particularly it is selected from the group consisting of saccharin, aspartame, cyclamate, sucralose, saccharine, stevia, rebauasdioside A, neotame, acesulfame K, sucrose, glucose fructose and sorbitol, more particularly it is selected from the group consisting of fructose and stevia.

In a further embodiment the sweetener is selected from the group consisting of sucrose, fructose, and stevia. In a further embodiment, the sweetener comprises a purified stevia extract having a high purity combination of nine sweet steviol glycosides found within the stevia leaf. The purified stevia extract may be represented by high purity combination of nine sweet steviol glycosides found within the stevia leaf wherein Reb A accounts for over half of the final composition (e.g., SG95 sold by PureCircle).

In one embodiment, Naringenin significantly increases the sweetness intensity of a lemon flavored bottled water containing 300 ppm of Steviol Glycosides (SG 95 from PureCircle) without significantly impacting any of the other flavor attributes. Moreover, the addition of 125 ppm Naringenin can maintain the sweetness intensity of a lemon flavored beverage having a 50 ppm reduced level of SG95. No maksing of bitterness was observed.

In another embodiment, 125 ppm Naringenin significantly increases the sweetness of 375 ppm Steviol Glycosides (SG95 from PureCircle) for example in a Strawberry flavored bottled water while also increasing the overall mouth-feel intensity, and lingering sweetness intensity. Moreover, the addition of 150 ppm Naringenin allows one to maintain the sweetness intensity of the beverage while reducing the level of Steviol glycosides (SG95). This allows one to create a beverage with reduced lingering sweetness and licorice aftertaste, providing a more pleasant tasting beverage. No masking of bitterness was observed.

In another embodiment, the addition from 10 to 25 ppm Naringenin to 150 ppm Reb.A solution does not bring any sweetness. In another embodiment, the addition of 50 ppm Naringenin to 150 ppm Reb.A solution enhances the sweetness intensity to 4% sucrose level.

In a further embodiment, the addition from 10 to 75 ppm Naringenin to a 300 ppm Reb.A solution has a 17% of sweetness enhancement effect. In another aspect, the addition of 100 and 150 ppm Naringenin to 300 ppm Reb.A solution has a small loss of sweetness (8%). In one embodiment provided herein is a food or beverage comprising from about 10 to 75 ppm Naringenin to up about 300 ppm Reb A.

In one embodiment, the addition of 10-25 ppm Naringenin to 150 ppm steviol glycosides (e.g., SG95) doesn't have any effect on sweetness. In another embodiment, the addition from from 50 to 150 ppm Naringenin to 150 ppm steviol glycosides (e.g., SG95) solution has a sweetness enhancement effect.

In another embodiment, the addition of 10-25 ppm Naringenin to 300 ppm steviol glycosides (e.g., PureCircle SG95) appears not to have any effect on sweetness. On the other hand, the addition of 50 to 150 ppm Naringenin to 300 ppm SG95 solution has a sweetness enhancement effect.

The compositions and methods provided herein have use in food or beverage products. When the food product is a particulate or powdery food, the dry particles may easily be added thereto by dry-mixing. Typical food products are selected from the group consisting of an instant soup or sauce, a breakfast cereal, a powdered milk, a baby food, a powdered drink, a powdered chocolate drink, a spread, a powdered cereal drink, a chewing gum, an effervescent tablet, a cereal bar, and a chocolate bar. The powdered foods or drinks may be intended to be consumed after reconstitution of the product with water, milk and/or a juice, or another aqueous liquid.

The food product may be selected from the group consisting of condiments, baked goods, powdery food, bakery filings and Fluid dairy products.

Condiments include, without limitation, ketchup, mayonnaise, salad dressing, Worcestershire sauce, fruit-flavored sauce, chocolate sauce, tomato sauce, chili sauce, and mustard.

Baked goods include, without limitation, cakes, cookies, pastries, breads, donuts and the like.

Bakery fillings include, without limitation, low or neutral pH fillings, high, medium or low solids fillings, fruit or milk based (pudding type or mousse type) fillings, hot or cold make-up fillings and nonfat to full-fat fillings.

Fluid dairy products include, without limitation, non-frozen, partially frozen and frozen fluid dairy products such as, for example, milks, ice creams, sorbets and yogurts.

Beverage products include, without limitation, carbonated soft drinks, including cola, lemon-lime, root beer, heavy citrus ("dew type"), fruit flavored and cream sodas; powdered soft drinks, as well as liquid concentrates such as fountain syrups and cordials; coffee and coffee-based drinks, coffee substitutes and cereal-based beverages; teas, including dry mix products as well as ready-to-drink teas (herbal and tealeaf based); fruit and vegetable juices and juice flavored beverages as well as juice drinks, nectars, concentrates, punches and "ades"; sweetened and flavored waters, both carbonated and still; sport/energy/health drinks; alcoholic beverages plus alcohol-free and other low-alcohol products including beer and malt beverages, cider, and wines (still, sparkling, fortified wines and wine coolers); other beverages processed with heating (infusions, pasteurization, ultra high temperature, ohmic heating or commercial aseptic sterilization) and hot-filled packaging; and cold-filled products made through filtration or other preservation techniques. SG95 is a natural, high purity combination of nine sweet steviol glycosides found within the stevia leaf. Reb A accounts for over half of the final composition.

The below examples are illustrative only and are not meant to limit the claims or embodiments described herein.

### EXAMPLES

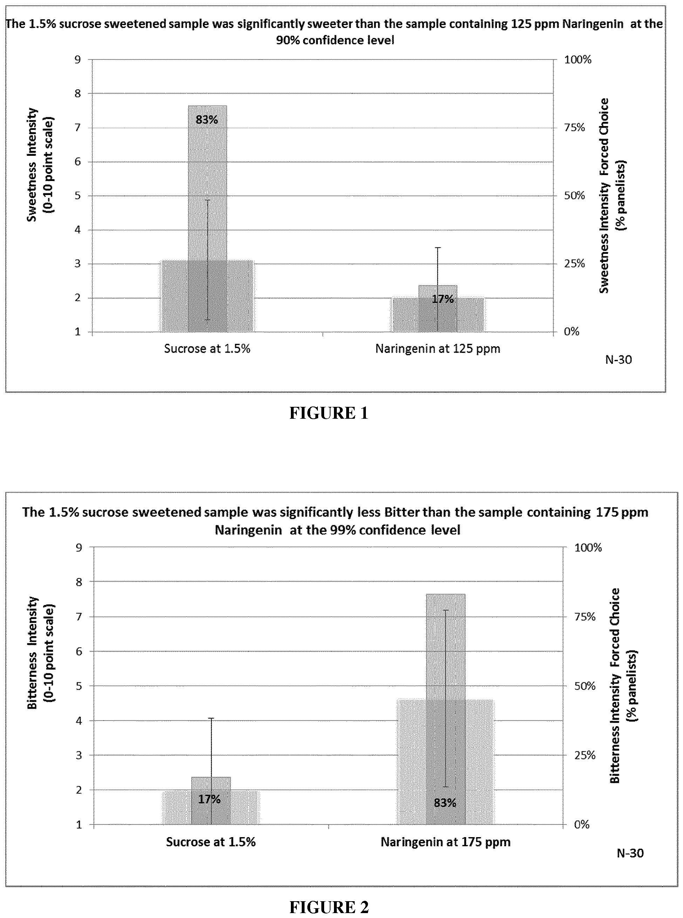

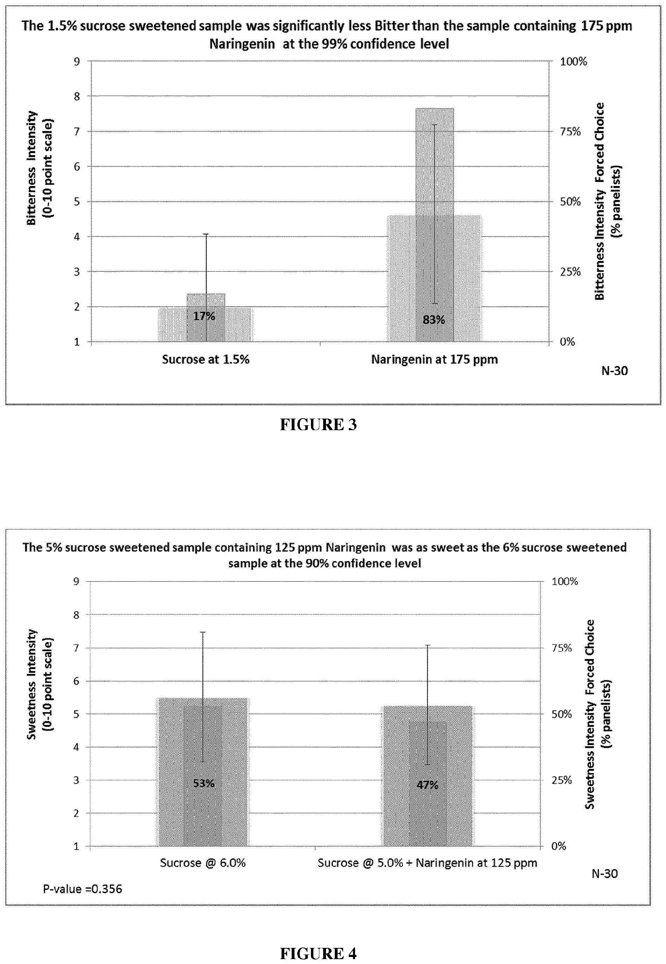

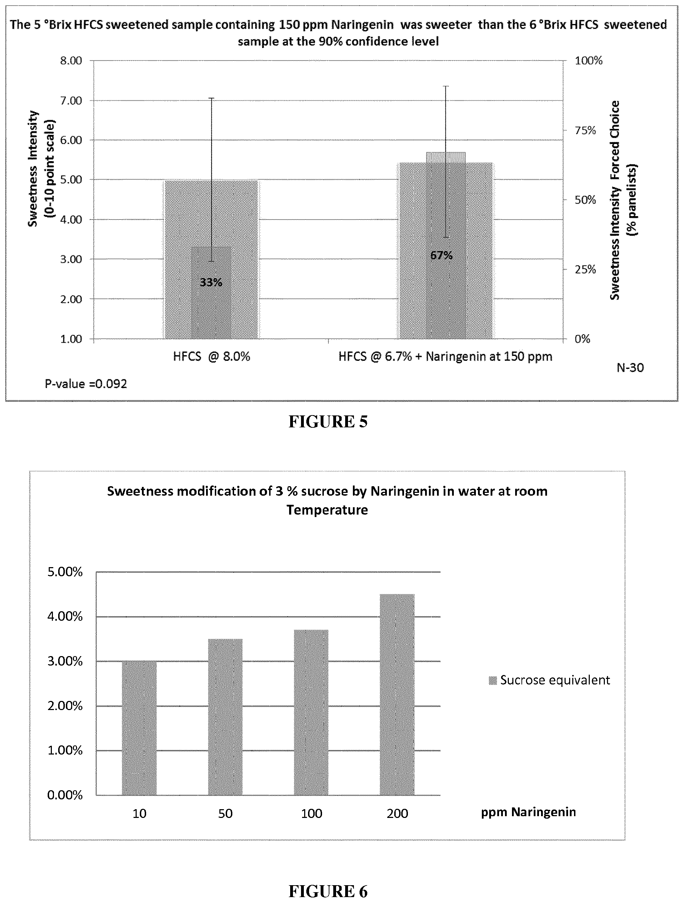

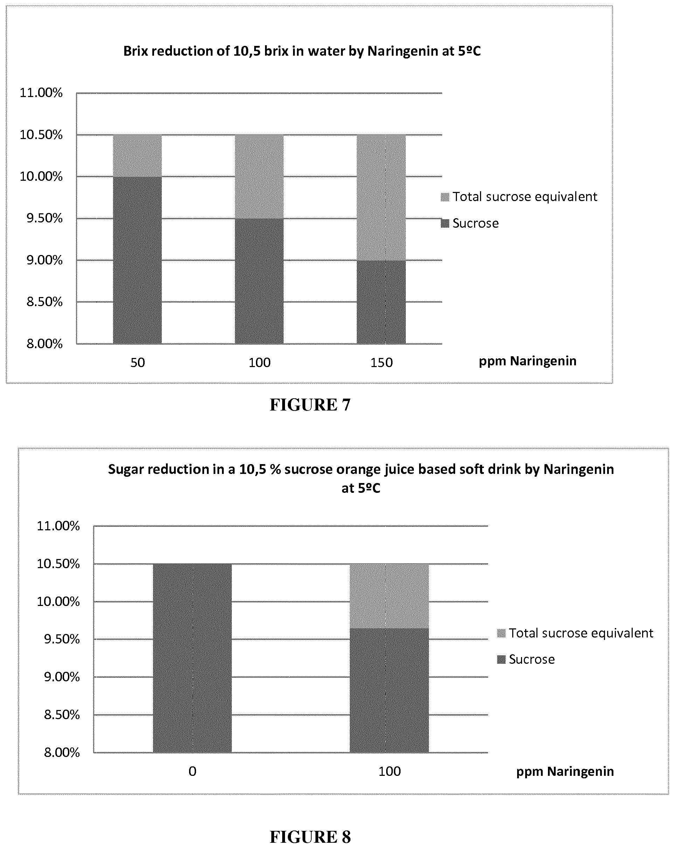

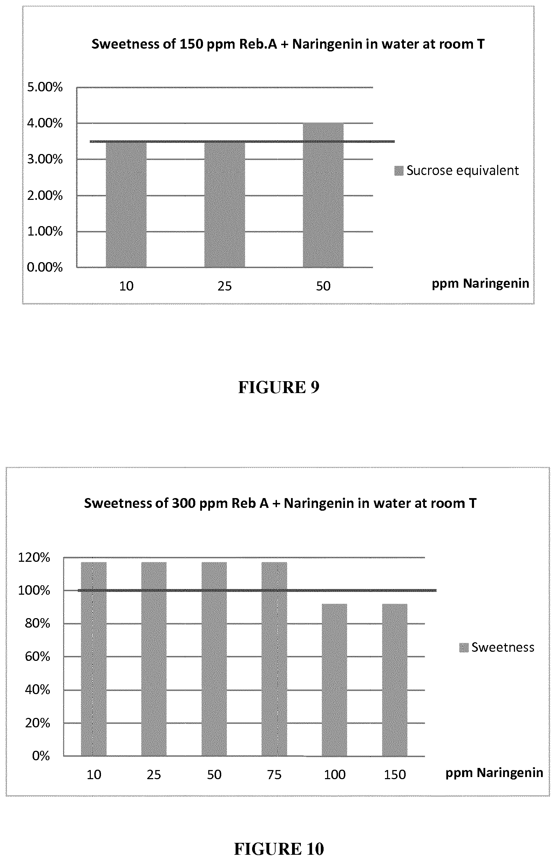

**Example 1.** Two samples were prepared. The first sample comprised Naringenin at 125 ppm in water. The second sample comprised Sucrose at 1.5% in water. Both samples were tested for sweetness intensity by thirty participants. The data displayed in Figure 1 shows that Naringenin is essentially not sweet on its own.
   The test was repeated (thirty participants) with 175 ppm Naringenin and again the data show that Naringenin is essentially not sweet on its own (Figure 2) at this concentration. This sample was also tested for bitterness and the data displayed in Figure 3 show that Naringenin is bitter at 175 ppm as compared to the sucrose sample.
**Example 2.** Two samples were prepared. The first sample was a 6% sucrose solution in water. The second sample was a 5% sucrose solution in water with 125 ppm Naringenin. The samples were tested for sweetness intensity by thirty participants. The data displayed in Figure 4 show that the sample with Naringenin enhanced the sweetness of the 5% solution to about the same level of sweetness as the 6% sucrose solution without Naringenin.
**Example 3.** Two samples were prepared. The first sample was a 6 °Brix high fructose corn syrup (HFCS) solution. The second sample was a 5 °Brix HFCS with 125 ppm Naringenin. The samples were tested for sweetness intensity by thirty participants. The data displayed in Figure 5 show that the sample with the Naringenin and 5 °Brix HFCS had essentially the same sweetness intensity as the sample without the Naringenin.
**Example 4.** Three samples were prepared. The first sample was a 3 ppm purified stevia extract (SG 95 from PureCircle) I lemon flavored bottled water. The second sample contained 300 ppm SG 95 and 125 ppm Naringenin in lemon flavored bottled water. The third sample contained 250 ppm SG 95 and 125 ppm Naringenin in lemon flavored bottled water. The samples were tested by thirty-one participants for lemon flavor, sweetness, overall mouth-feel, licorice intensity, bitterness, fresh lemon, peely, lingering sweetness and lingering licorice. The data as displayed in Figure 13 show that Naringenin significantly increases the sweetness intensity of a lemon flavored bottled water containing 300 ppm of Steviol Glycosides (SG 95 from PureCircle) without significantly impacting any of the other flavor attributes. Moreover, example 4 shows that the addition of 125 ppm Naringenin can maintain the sweetness intensity of a lemon flavored beverage having a 50 ppm reduced level of SG95. No masking of bitterness was observed.
**Example 5.** Three samples were prepared. The first sample was a strawberry flavored bottled water sample containing 375 ppm SG 95. The second sample was a strawberry flavored bottled water containing 375 ppm SG 95 plus 125 ppm Naringenin. The third sample was a strawberry flavored bottled water sample containing 300 ppm SG 95 and 150 ppm Naringenin. The samples were tested by thirty participants for strawberry flavor, sweetness, overall mouth-feel, licorice intensity, bitterness, cooked, jammy, candied, lingering sweetness and lingering licorice. The data displayed in Figure 14 show that 125 ppm Naringenin significantly increases the sweetness of 375 ppm Steviol Glycoside (SG95 from PureCircle) in a Strawberry flavored bottled water while also increasing the overall mouth-feel intensity, and lingering sweetness intensity. Moreover, the addition of 150 ppm Naringenin allows one to maintain the sweetness intensity of the beverage while reducing the level of Steviol glycosides (SG95). This allows one to create a beverage with reduced lingering sweetness and licorice aftertaste, providing a more pleasant tasting beverage. No masking of bitterness was observed.
**Example 6.** Four samples were prepared and compared with a 3 % sucrose solution in water. The first sample comprised sucrose at 3% with 10 ppm Naringenin (Interquim, S.A). The second sample was a 3% sucrose solution with 50 ppm Naringenin. The third sample was a 3% sucrose solution with 100 ppm Naringenin. The fourth sample was a 3% sucrose solution with 200 ppm Naringenin. All samples were tested for sweetness intensity at room temperature by four experts especially trained to evaluate sweet products. The data displayed in Figure 6 shows that the addition from 50 to 200 ppm Naringenin to 3% sucrose solution enhances the sweetness intensity of 3% sucrose in water.
**Example 7:** Three samples were prepared and compared with a 10,5 % sucrose solution in water. The first sample comprised sucrose at 10% with 50 ppm Naringenin. The second sample was a 9.5% sucrose solution with 100 ppm Naringenin. The third sample was a 9% sucrose solution with 150 ppm Naringenin. All samples were tested for sweetness intensity at the refrigeration Temperature by four experts especially trained to evaluate sweet products. The data displayed in Figure 7 shows that the addition from 50 to 150 ppm of Naringenin to 10, 9.5 and 9% sucrose solutions in water maintains the same sweetness intensity level as a 10,5% sucrose solution.
**Example 8.** Two samples were prepared. The first sample was a 10,5% sucrose orange juice based soft drink. The second sample was a 9,65% sucrose orange juice based SD with 100 ppm Naringenin. The samples were tested for sweetness intensity at refrigeration Temperature by four experts especially trained to evaluate sweet products. The data displayed in Figure 8 shows that the sample with the Naringenin and 9,65% sucrose had essentially the same sweetness intensity as the sample with 10,5% sucrose.
**Example 9:** Three samples were prepared and compared with 150 ppm Reb.A (PureCircle) in water; equisweet to 3,5% sucrose (in red, Figure 9). The first sample comprised 150 ppm Reb.A with 10 ppm Naringenin. The second sample was a 150 ppm Reb.A with 25 ppm Naringenin. The third sample comprised 150 ppm Reb.A with 50 ppm Naringenin. All samples were tested for sweetness intensity at room Temperature by four experts especially trained to evaluate sweet products. The data displayed in Figure 9 shows that the addition from 10 to 25 ppm Naringenin to 150 ppm Reb.A solution doesn't bring any sweetness. On the other hand, the addition of 50 ppm Naringenin to 150 ppm Reb.A solution enhances the sweetness intensity to 4% sucrose level.
**Example 10:** Six samples were prepared and compared with 300 ppm Reb.A in water; 100% sweetness level (in red, Figure 10). The first sample comprised 300 ppm Reb.A with 10 ppm Naringenin. The second sample was a 300 ppm Reb.A with 25 ppm Naringenin. The third sample was a 300 ppm Reb.A with 50 ppm Naringenin. The fourth sample comprised 300 ppm Reb.A with 75 ppm Naringenin. The fifth sample was a 300 ppm Reb.A with 100 ppm Naringenin. The sixth sample was a 300 ppm Reb.A with 150 ppm Naringenin. All samples were tested for sweetness intensity at room Temperature by four experts especially trained to evaluate sweet products. The data displayed in Figure 10 shows that the addition from 10 to 75 ppm Naringenin to 300 ppm Reb.A solution has a 17% of sweetness enhancement effect; all of them were equisweet to 350 ppm Reb.A in water. On the other hand, the addition of 100 and 150 ppm Naringenin to 300 ppm Reb.A solution has a small loss of sweetness (8%).
**Example 11:** Six samples were prepared and compared with 150 ppm SG95 (PureCircle) in water; 100% sweetness level (in red, Figure 11). The first sample comprised 150 ppm SG95 with 10 ppm Naringenin. The second sample was a 150 ppm SG95 with 25 ppm Naringenin. The third sample was a 150 ppm SG95 with 50 ppm Naringenin. The fourth sample comprised 150 ppm SG95 with 75 ppm Naringenin. The fifth sample was a 150 ppm SG95 with 100 ppm Naringenin. The sixth sample was a 150 ppm SG95 with 150 ppm Naringenin. All samples were tested for sweetness intensity at room Temperature by four experts especially trained to evaluate sweet products. The data displayed in Figure 11 shows that the addition of 10-25 ppm Naringenin to 150 ppm SG95 doesn't have any effect in sweetness. On the other hand, the addition from from 50 to 150 ppm Naringenin to 150 ppm SG95 solution has a sweetness enhancement effect.
**Example 12:** Six samples were prepared and compared with 300 ppm SG95 in water; 100% sweetness level (in red, Figure 12). The first sample comprised 300 ppm SG95 with 10 ppm Naringenin. The second sample was a 300 ppm SG95 with 25 ppm Naringenin. The third sample was a 300 ppm SG95 with 50 ppm Naringenin. The fourth sample comprised 300 ppm SG95 with 75 ppm Naringenin. The fifth sample was a 300 ppm SG95 with 100 ppm Naringenin. The sixth sample was a 300 ppm SG95 with 150 ppm Naringenin. All samples were tested for sweetness intensity at room Temperature, by four experts especially trained to evaluate sweet products. The data displayed in Figure 12 shows that the addition of 10-25 ppm Naringenin to 300 ppm SG95 doesn't have any effect in sweetness. On the other hand, the addition from 50 to 150 ppm Naringenin to 300 ppm SG95 solution has a sweetness enhancement effect.

## Claims

1. A method of enhancing the sweetness of sweetener in a food or beverage product comprising adding Naringenin to the product in an amount of 30 ppm to 200 ppm by weight of the total weight of the product wherein the Naringenin does not block the bitter taste of the product when compared to the food or beverage without Naringenin.

2. The method as recited in claim 1 wherein the food or beverage product comprises Naringenin in an amount of 30 to 75 ppm or 125 to 200 ppm by weight of the total weight of the product.

3. The method as recited in any one of claim 1 to 2 wherein the sweetener is selected from the group consisting of the common saccharide sweeteners, namely sucrose, fructose, such as D-fructose, glucose, such as D-glucose, and sweetener compositions comprising natural sugars, namely corn syrup including high fructose corn syrup or other syrups or sweetener concentrates derived from natural fruit and vegetable sources, semisynthetic "sugar alcohol" sweeteners, namely erythritol, isomalt, lactitol, mannitol, sorbitol, xylitol, maltodextrin, glycerol, threitol, arabitol, ribitol and dulcitol, and artificial sweeteners namely miraculin, aspartame, superaspartame, saccharin, saccharin-sodium salt, acesulfame-K,cyclamate, sodium cyclamate, and alitame and other sweeteners, namely trehalose, melizitose, melibiose, raffinose, palatinose, lactulose, cyclamic acid, mogroside, tagatose, such as D-tagatose, maltose, galactose, such as D-galactose, L-rhamnose, D-sorbose, maunose, such as D-maunose, lactose, L-arabinose, D-ribose, D-glyceraldehyde, curculin, brazzein, mogroside, Neohesperidin dihydrochalcone (NHDC), neotame, D-tryptophan, D-Ieucine, D-threonine, glycine, D-asparagine, D-phenylalanine, L-proline, maltitol, hydrogenated glucose syrup (HGS), magap, sucralose, lugduname, sucrononate, sucrooctate, monatin, phyllodulcin, hydrogenated starch hydrolysate (HSH), stevioside, rebaudioside A and other sweet Stevia based glycosides, lo han guo, thaumatin, monellin, carrelame and other guanidine-based sweeteners.

4. The method as recited in any one of claims 1 to 3 wherein the product or beverage is not coffee.

5. A food or beverage product comprising Naringenin in an amount of from 30 to 200 ppm, by weight, of the total weight of the product, and a sweetener wherein the Naringenin does not block the bitter taste of the product.

6. A food or beverage product according to claim 5, wherein the product is not a product selected from coffee or tea.

7. A food or beverage product as recited in claim 5 or 6 comprising Narengenin in an amount of 30 to 75 ppm or 125 to 200 ppm by weight of the total weight of the product.

8. The food or beverage as recited in claim 5 wherein the sweetener is selected from the group consisting of the common saccharide sweeteners, namely sucrose, fructose, such as D-fructose, glucose, such as D-glucose, and sweetener compositions comprising natural sugars, namely corn syrup including high fructose corn syrup or other syrups or sweetener concentrates derived from natural fruit and vegetable sources, semisynthetic "sugar alcohol" sweeteners, namely erythritol, isomalt, lactitol, mannitol, sorbitol, xylitol, maltodextrin, glycerol, threitol, arabitol, ribitol and dulcitol, and artificial sweeteners, namely miraculin, aspartame, superaspartame, saccharin, saccharin-sodium salt, acesulfame-K, cyclamate, sodium cyclamate, and alitame and other sweeteners, namely trehalose, melizitose, melibiose, raffinose, palatinose, lactulose, cyclamic acid, mogroside, tagatose, such as D-tagatose, maltose, galactose, such as D-galactose, L-rhamnose, D-sorbose, maunose, such as D-maunose, lactose, L-arabinose, D-ribose, D-glyceraldehyde, curculin, brazzein, mogroside, Neohesperidin dihydrochalcone (NHDC), neotame, D-tryptophan, D-Ieucine, D-threonine, glycine, D-asparagine, D-phenylalanine, L-proline, maltitol, hydrogenated glucose syrup (HGS), magap, sucralose, lugduname, sucrononate, sucrooctate, monatin, phyllodulcin, hydrogenated starch hydrolysate (HSH), stevioside, rebaudioside A and other sweet Stevia based glycosides, lo han guo, thaumatin, monellin, carrelame and other guanidine-based sweeteners.

## Patentansprüche

1. Verfahren zum Verbessern der Süßkraft eines Süßungsmittels in einem Nahrungsmittel- oder Getränkeprodukt, umfassend Zugeben von Naringenin zu dem Produkt in einer Menge von 30 ppm bis 200 ppm, auf das Gewicht bezogen, des Gesamtgewichts des Produkts, wobei das Naringenin den bitteren Geschmack des Produkts im Vergleich mit dem Nahrungsmittel oder Getränk ohne Naringenin nicht blockiert.

2. Verfahren wie in Anspruch 1 aufgeführt, wobei das Nahrungsmittel- oder Getränkeprodukt Naringenin in einer Menge von 30 bis 75 ppm oder 125 bis 200 ppm, auf das Gewicht bezogen, des Gesamtgewichts des Produkts umfasst.

3. Verfahren wie in einem der Ansprüche 1 bis 2 aufgeführt, wobei das Süßungsmittel aus der Gruppe ausgewählt ist bestehend aus den gewöhnlichen Saccharidsüßungsmitteln, nämlich Sucrose, Fructose, wie D-Fructose, Glucose, wie D-Glucose, und Süßungsmittelzusammensetzungen umfassend natürliche Zucker, nämlich Maissirup einschließlich Maissirup mit hohem Fructosegehalt oder andere Sirupe oder Süßungsmittelkonzentrate, die von natürlichen Obst- und Gemüsequellen abgeleitet sind, halbsynthetische "Zuckeralkohol-" Süßungsmittel, nämlich Erythrit, Isomalt, Lactitol, Mannit, Sorbit, Xylit, Maltodextrin, Glycerin, Threit, Arabit, Ribit und Dulcit, und künstliche Süßungsmittel, nämlich Miraculin, Aspartam, Superaspartam, Saccharin, Saccharin-Natriumssalz, Acesulfam-K, Cyclamat, Natriumcyclamat und Alitam und andere Süßungsmittel, nämlich Trehalose, Melizitose, Melibiose, Raffinose, Palatinose, Lactulose, Cyclaminsäure, Mogrosid, Tagatose wie D-Tagatose, Maltose, Galactose, wie D-Galactose, L-Rhamnose, D-Sorbose, Maunose, wie D-Maunose, Lactose, L-Arabinose, D-Ribose, D-Glyceraldehyd, Curculin, Brazzein, Mogrosid, Neohesperidindihydrochalcon (NHDC), Neotam, D-Tryptophan, D-Leucin, D-Threonin, Glycin, D-Asparagin, D-Phenylalanin, L-Prolin, Maltitol, hydrierten Glucosesirup (HGS), Magap, Sucralose, Lugdunam, Sucrononat, Sucrooctat, Monatin, Phyllodulcin, hydriertes Stärkehydrolysat (HSH), Steviosid, Rebaudiosid A und andere süße Glycoside auf der Basis von Stevia, lo han guo, Thaumatin, Monellin, Carrelam und andere Süßungsmittel auf der Basis von Guanidin.

4. Verfahren wie in einem der Ansprüche 1 bis 3 aufgeführt, wobei das Produkt oder das Getränk nicht Kaffee ist.

5. Nahrungsmittel- oder Getränkeprodukt umfassend Naringenin in einer Menge von 30 bis 200 ppm, auf das Gewicht bezogen, des Gesamtgewichts des Produkts, und ein Süßungsmittel, wobei das Naringenin den bitteren Geschmack des Produkts nicht blockiert.

6. Nahrungsmittel- oder Getränkeprodukt nach Anspruch 5, wobei das Produkt kein Produkt ausgewählt von Kaffee oder Tee ist.

7. Nahrungsmittel- oder Getränkeprodukt wie in Anspruch 5 oder 6 aufgeführt, umfassend Narengenin in einer Menge von 30 bis 75 ppm oder 125 bis 200 ppm, auf das Gewicht bezogen, des Gesamtgewichts des Produkts.

8. Nahrungsmittel- oder Getränkeprodukt wie in Anspruch 5 aufgeführt, wobei das Süßungsmittel aus der Gruppe ausgewählt ist bestehend aus den gewöhnlichen Saccharidsüßungsmitteln, nämlich Sucrose, Fructose, wie D-Fructose, Glucose, wie D-Glucose, und Süßungsmittelzusammensetzungen umfassend natürliche Zucker, nämlich Maissirup einschließlich Maissirup mit hohem Fructosegehalt oder andere Sirupe oder Süßungsmittelkonzentrate, die von natürlichen Obst- und Gemüsequellen abgeleitet sind, halbsynthetische "Zuckeralkohol-" Süßungsmittel, nämlich Erythrit, Isomalt, Lactitol, Mannit, Sorbit, Xylit, Maltodextrin, Glycerin, Threit, Arabit, Ribit und Dulcit, und künstliche Süßungsmittel, nämlich Miraculin, Aspartam, Superaspartam, Saccharin, Saccharin-Natriumssalz, Acesulfam-K, Cyclamat, Natriumcyclamat und Alitam und andere Süßungsmittel, nämlich Trehalose, Melizitose, Melibiose, Raffinose, Palatinose, Lactulose, Cyclaminsäure, Mogrosid, Tagatose wie D-Tagatose, Maltose, Galactose, wie D-Galactose, L-Rhamnose, D-Sorbose, Maunose, wie D-Maunose, Lactose, L-Arabinose, D-Ribose, D-Glyceraldehyd, Curculin, Brazzein, Mogrosid, Neohesperidindihydrochalcon (NHDC), Neotam, D-Tryptophan, D-Leucin, D-Threonin, Glycin, D-Asparagin, D-Phenylalanin, L-Prolin, Maltitol, hydrierten Glucosesirup (HGS), Magap, Sucralose, Lugdunam, Sucrononat, Sucrooctat, Monatin, Phyllodulcin, hydriertes Stärkehydrolysat (HSH), Steviosid, Rebaudiosid A und andere süße Glycoside auf der Basis von Stevia, lo han guo, Thaumatin, Monellin, Carrelam und andere Süßungsmittel auf der Basis von Guanidin.

## Revendications

1. Procédé destiné à renforcer le goût sucré d'un édulcorant dans un produit alimentaire ou de boisson comprenant l'ajout de la naringénine au produit en une quantité de 30 ppm à 200 ppm en poids du poids total du produit, dans lequel la naringénine ne bloque pas le goût amer du produit lorsque comparé à l'aliment ou à la boisson sans naringénine.

2. Procédé selon la revendication 1, dans lequel le produit alimentaire ou de boisson comprend de la naringénine en une quantité de 30 à 75 ppm ou de 125 à 200 ppm en poids du poids total du produit.

3. Procédé selon l'une quelconque des revendications 1 à 2, dans lequel l'édulcorant est sélectionné parmi le groupe consistant en des édulcorants communs à base de saccharides, nommément le saccharose, le fructose tel que le D-fructose, le glucose tel que le D-glucose, et des compositions édulcorantes comprenant des sucres naturels, nommément le sirop de maïs y compris le sirop de maïs à haute teneur en fructose ou d'autres sirops ou concentrés édulcorants dérivés de sources naturelles de fruits et de légumes, des édulcorants semisynthétiques type sucre-alcool, nommément l'érythritol, l'isomalt, le lactitol, le mannitol, le sorbitol, le xylitol, la maltodextrine, le glycérol, le thréitol, l'arabitol, le ribitol et le dulcitol, et des édulcorants artificiels, nommément la miraculine, l'aspartame, le superaspartame, la saccharine, le sel sodique de saccharine, l'acésulfame-K, le cyclamate, le cyclamate de sodium et l'alitame et d'autres édulcorants, nommément le tréhalose, le mélizitose, le mélibiose, le raffinose, le palatinose, le lactulose, l'acide cyclamique, le mogroside, le tagatose tel que le D-tagatose, le maltose, le galactose tel que le D-galactose, le L-rhamnose, le D-sorbose, le maunose tel que le D-maunose, le lactose, le L-arabinose, le D-ribose, le D-glycéraldéhyde, la curculine, la brazzéine, le mogroside, la dihydrochalcone de néohespéridine (NHDC), le néotame, le D-tryptophane, la D-leucine, la D-théonine, la glycine, la D-asparagine, la D-phénylalanine, la L-proline, le maltitol, le sirop de glucose hydrogéné (HGS), le magap, le sucralose, le lugduname, le sucrononate, le sucrooctate, la monatine, la phyllodulcine, l'hydrolysat d'amidon hydrogéné (HSH), le stévioside, le rébaudioside A et d'autres glucosides à saveur sucrée à base de Stevia, le fruit du arhat, la thaumatine, la monelline, le carrélame et d'autres édulcorants à base de guanidine.

4. Procédé selon l'une quelconque des revendications 1 à 3, dans lequel le produit ou la boisson n'est pas du café.

5. Produit alimentaire ou de boisson comprenant de la naringénine en une quantité de 30 à 200 ppm en poids du poids total du produit, et un édulcorant dans lequel la naringénine ne bloque pas le goût amer du produit.

6. Produit alimentaire ou de boisson selon la revendication 5, où le produit n'est pas un produit sélectionné parmi le café ou le thé.

7. Produit alimentaire ou de boisson selon la revendication 5 ou 6, comprenant de la naringénine en une quantité de 30 à 75 ppm ou de 125 à 200 ppm en poids du poids total du produit.

8. Aliment ou boisson selon la revendication 5, dans lequel l'édulcorant est sélectionné parmi le groupe consistant en des édulcorants communs à base de saccharides, nommément le saccharose, le fructose tel que le D-fructose, le glucose tel que le D-glucose, et des compositions édulcorantes comprenant des sucres naturels, nommément le sirop de maïs y compris le sirop de maïs à haute teneur en fructose ou d'autres sirops ou concentrés édulcorants dérivés de sources naturelles de fruits et de légumes, des édulcorants semisynthétiques type sucre-alcool, nommément l'érythritol, l'isomalt, le lactitol, le mannitol, le sorbitol, le xylitol, la maltodextrine, le glycérol, le thréitol, l'arabitol, le ribitol et le dulcitol, et des édulcorants artificiels, nommément la miraculine, l'aspartame, le superaspartame, la saccharine, le sel sodique de saccharine, l'acésulfame-K, le cyclamate, le cyclamate de sodium et l'alitame et d'autres édulcorants, nommément le tréhalose, le mélizitose, le mélibiose, le raffinose, le palatinose, le lactulose, l'acide cyclamique, le mogroside, le tagatose tel que le D-tagatose, le maltose, le galactose tel que le D-galactose, le L-rhamnose, le D-sorbose, le maunose tel que le D-maunose, le lactose, le L-arabinose, le D-ribose, le D-glycéraldéhyde, la curculine, la brazzéine, le mogroside, la dihydrochalcone de néohespéridine (NHDC), le néotame, le D-tryptophane, la D-leucine, la D-théonine, la glycine, la D-asparagine, la D-phénylalanine, la L-proline, le maltitol, le sirop de glucose hydrogéné (HGS), le magap, le sucralose, le lugduname, le sucrononate, le sucrooctate, la monatine, la phyllodulcine, l'hydrolysat d'amidon hydrogéné (HSH), le stévioside, le rébaudioside A et d'autres glucosides à saveur sucrée à base de Stevia, le fruit du arhat, la thaumatine, la monelline, le carrélame et d'autres édulcorants à base de guanidine.
